# EUROPÄISCHE PATENTANMELDUNG

(11) **EP 0 736 763 A1**
(43) Veröffentlichungstag der Anmeldung: **09.10.1996**
(21) Anmeldenummer: 96101156.6
(22) Anmeldetag: 27.01.1996
(51) Int. Cl.: G01N 1/26, B08B 9/46, H01J 49/04

(54) **Vorrichtung zum Prüfen von Behältern**

(30) Priorität: 03.04.1995 CH 927/95; 07.04.1995 CH 999/95
(71) Anmelder: ELPATRONIC AG, CH-6303 Zug (CH)
(72) Erfinder: Gysi, Peter, CH-5454 Bellikon (CH); Huesser, Theo, CH-8964 Rudolfstetten (CH); Jasny, Thaddäus, D-65189 Wiesbaden (DE); Rosatzin, Martin, Dr., CH-8953 Dietikon (CH)

(57) **Zusammenfassung**

Bei einem Verteilkopf (1) für die Verteilung von aus zu untersuchenden Flaschen entnommenen Gasproben an die Anschlüsse (14,15) zweier Massenspektrometer sind verschiedene Kanalführungen der Zuleitungskanäle (19,20,23,24,23',24') möglich. Die verschiedenen Kanalführungen sind in einem auswechselbaren Stopfen (21,21',22) vorgesehen. Dadurch kann auf einfache Weise der Betrieb mit zwei Massenspektrometern auf den Betrieb mit einem Massenspektrometer umgestellt werden, was die einfache Wartung der Massenspektrometer erleichtert.

## Beschreibung

Die Erfindung betrifft eine Vorrichtung zum Prüfen von Behältern, insbesondere Flaschen, gemäss dem Oberbegriff des Patentanspruchs 1, bzw. gemäss dem Oberbegriff des Patentanspruchs 6.

Eine derartige Vorrichtung ist aus EP-A-0 579 952 bekannt. Dort sind die von den Probeentnahmeorganen zu dem Verteilkopf laufenden Leitungen in zwei konzentrischen Kreisen am beweglichen Teil des Verteilkopfes angeschlossen. Am feststehenden Teil sind zwei Prüfgeräte, in der Regel Massenspektrometer, angeschlossen, von denen jedes einem der Anschlusskreise fest zugeordnet ist. Bei der aus EP-A-0 579 952 bekannten Vorrichtung ist ein Betrieb nur mit beiden Massenspektrometern möglich; muss eines der Geräte gewartet werden, so steht die Vorrichtung still.

Der Erfindung liegt die Aufgabe zugrunde, diesen Nachteil zu vermeiden. Dies wird bei einer Vorrichtung der eingangs genannten Art mit den kennzeichnenden Merkmalen des Anspruchs 1 gelöst; eine alternative Lösung ergibt sich mit den kennzeichnenden Merkmalen des Anspruchs 6.

Dadurch, dass wahlweise nur ein Massenspektrometer mit beiden Anschlusskreisen verbindbar ist, ergibt sich die Möglichkeit, alle Flaschen mit dem einen Prüfgerät bzw. Massenspektrometer zu prüfen. Sofern ein zweites Massenspektrometer vorgesehen ist, kann dieses gewartet werden, worauf auf einfache Weise nach der Wartung wieder auf einen Betrieb mit zwei Massenspektrometern umgestellt werden kann. Sofern gewünscht, kann auch längere Zeit mit nur einem Massenspektrometer gefahren werden und erst bei einer notwendigen Erhöhung des Flaschendurchsatzes kann auf einfache Weise ein zweites Massenspektrometer eingesetzt werden.

Im folgenden werden Ausführungsbeispiele der Erfindung anhand der Zeichnungen näher erläutert. Dabei zeigt
Figur 1 eine teilweise geschnittene Ansicht eines Verteilkopfes;
Figur 2 eine Draufsicht auf die drehende Scheibe des Verteilkopfes;
Figur 3 eine Ansicht der feststehenden Scheibe des Verteilkopfes;
Figur 4 eine teilweise geschnittene Detailansicht des Verteilkopfes mit einer ersten Ausführungsform des Verteilstopfens;
Figur 5 eine teilweise geschnittene Detailansicht des Verteilkopfes mit einer zweiten Ausführungsform des Verteilstopfens;
Figur 6 eine teilweise Ansicht der feststehenden Scheibe für die Ausführungsbeispiele der Figuren 4 und 5;
Figur 7 eine Ausführungsform des feststehenden Teils des Verteilkopfes in teilweise geschnittener Darstellung;
Figur 8 eine Ansicht des feststehenden Teils des Verteilkopfes gemäss der Ausführung nach Figur 7; und
Figur 9 schematisch eine Ansicht einer weiteren Ausführungsform.

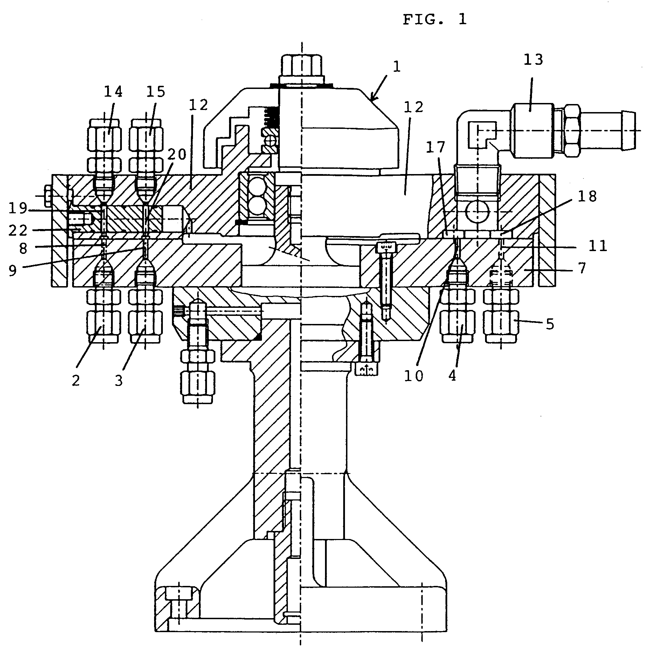

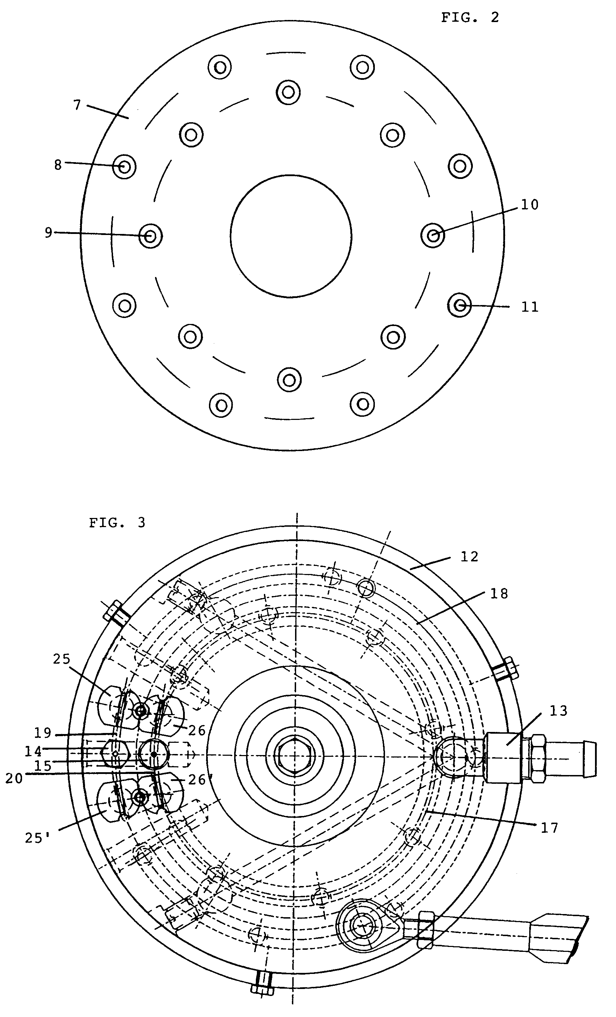

Figur 1 zeigt einen Verteilkopf für die aus den zu untersuchenden Flaschen entnommenen Gasproben, wie er grundsätzlich aus der EP-A-579 952 bekannt ist. Die Flaschen laufen in einem nicht dargestellten, unterhalb des Verteilkopfes angeordneten Karussell, und Probeentnahmeorgane tauchen in die Flaschen ein, um Gasproben zu entnehmen. Diese Gasproben gelangen über Leitungen und Anschlussstücke, von denen in Figur 1 vier Stück 2, 3, 4, 5 sichtbar sind, an die untere, mit dem Karussell mitdrehende Scheibe 7 des Verteilkopfes. Figur 2 zeigt eine Draufsicht auf diese Scheibe, woraus die Anordnung der Anschlussstücke bzw. der entsprechenden Kanäle (8,9,10,11 in Figur 1) in der Scheibe ersichtlich sind. Die obere, feststehende Scheibe 12 des Verteilkopfes weist einen Sauganschluss 13 auf, der mit einer nicht dargestellten Saugpumpe verbunden ist, und zwei Anschlussstücke 14 und 15, von welchen je eine Leitung zum jeweiligen Eingang zweier nicht dargestellter Massenspektrometer führt. Der Sauganschluss mündet in zwei konzentrische Saugkammern 17 und 18. In Figur 3 sind diese Saugkammern 17 und 18 gestrichelt dargestellt und es ist ersichtlich, wie der Sauganschluss an drei Stellen in die Kammern 17 und 18 mündet, was gegenüber einer einfachen Mündung bevorzugt ist. Die Saugkammern erstrecken sich fast entlang der ganzen Scheibe und lassen lediglich einen Sektor frei, wobei sich in diesem Sektor die Anschlussstücke 14 und 15 für die Massenspektrometer sowie zwei diesen Anschlussstücken zugeordnete Kanäle 19 und 20 erstrecken.

Die Funktion des Verteilkopfes ist dabei zunächst grundsätzlich gleich wie diejenige des aus der EP-A-0 579 925 bekannten Verteilkopfes. Jeweils zwei Anschlussstücke (2,3 in Figur) stehen über die Kanäle 19 und 20 in Verbindung mit den Anschlussstücken 14 und 15 bzw. mit den Massenspektrometern und die jeweilige Gasprobe erreicht die Massenspektrometer. Zur gleichen Zeit stehen alle anderen Anschlussstücke (4 und 5 in Figur 1) über die Kanäle 17 und 18 in Verbindung mit der Saugpumpe, so dass Gas aus den entsprechenden Flaschen zum Verteilkopf angesaugt wird. Bei der weiterdrehung der unteren Scheibe 7 gegenüber der feststehenden oberen Scheibe 12 ergibt sich dann, dass die nächsten beiden Anschlussstücke in Verbindung mit den Kanälen 19 und 20 gelangen und die Gasproben aus den zugehörigen Flaschen zu den Massenspektrometern usw. Der Verteilkopf sorgt damit dafür, dass immer zwei anliegende Gasproben zu den beiden Massenspektrometern durchgeschaltet werden, während aus den anderen Flaschen Gasproben angesaugt und somit zur Weiterleitung an die Massenspektrometer bereit gemacht werden.

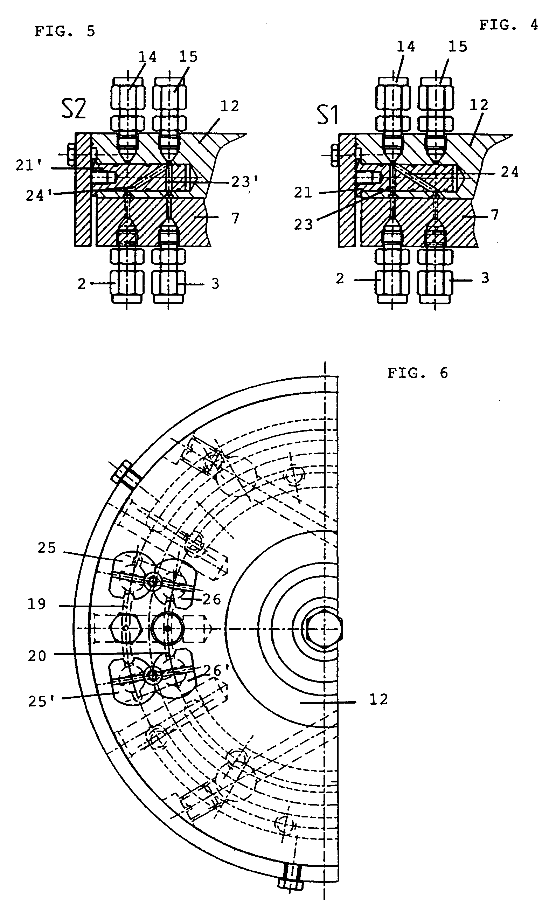

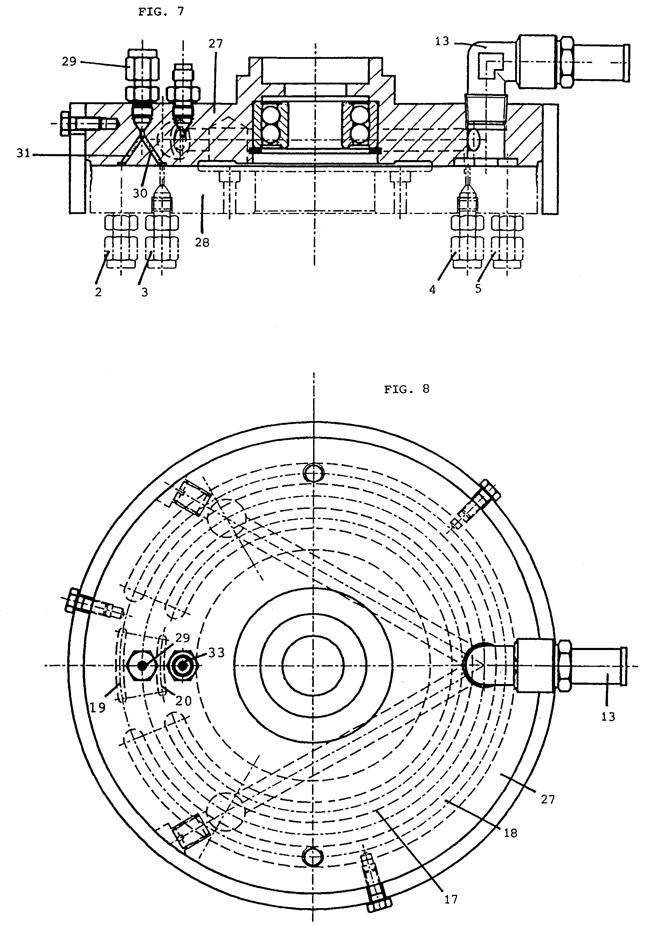

Im gezeigten Beispiel sind die Kanäle 19, 20 in einem auswechselbaren Stopfen 22 angeordnet, der seitlich in die feststehende Scheibe 12 eingesetzt ist. Der Stopfen 22 kann entnommen werden und durch einen Stopfen 21 ersetzt werden, der eine andere Anordnung von Kanälen 23, 24 aufweist, wie dies aus Figur 4 ersichtlich ist, welche eine Detailansicht des Verteilkopfes 1 zeigt und wobei gleiche Bezugsziffern wie bis anhin erwähnt gleiche Teile bezeichnen. Die Kanäle 23, 24 sind hier so ausgeführt, dass die Gasproben nur noch zu dem mit dem Anschlussstück 14 verbundenen Massenspektrometer gelangen. Das am Anschlussstück 15 angeschlossene Massenspektrometer bleibt somit ausser Funktion und kann gewartet oder repariert werden. Figur 5 zeigt eine andere Ausführungsform mit einem Stopfen 21' und den Kanälen 23' und 24', bei dem die Gasproben zu dem am Anschluss 15 angeschlossenen Massenspektrometer gelangen. Damit die Gasproben von den Anschlussstücken 2, 3 das Massenspektrometer nicht gleichzeitig erreichen, ist bei dem gezeigten Beispiel eine Verkürzung der Kanäle 19 und 20 nötig, so dass der winkelmässige Versatz der Anschlussstücke (vgl. Figur 2) zwischen den beiden konzentrischen Anschlusskreisen genügt, um ein sequentielles Zuführen der Gasproben von den beiden Anschlusskreisen an das eine Massenspektrometer zu gewährleisten. Figur 6 zeigt die Verkürzung der Kanäle 19, 20 mittels in der Scheibe 12 eingesetzten Bolzen 25, 26 bzw. 25', 26', welche jeweils einen Teil des Kanals 19 bzw. 20 bilden und durch Drehung um eine vertikale Achse (parallel zur Karussellachse) so gestellt werden können, dass der jeweilige Kanal 19 bzw. 20 länger oder kürzer wird. Figur 6 zeigt dabei die Stellung mit kurzen Kanälen, Figur 3 die Stellung mit langen Kanälen.

Figur 7 zeigt eine weitere Ausführungsform der Erfindung, wobei die Figur die obere, feststehende Scheibe 27 des Verteilkopfes in teilweise geschnittener Darstellung zeigt; die untere Scheibe 28 ist lediglich angedeutet. Gleiche Bezugsziffern wie beider vorhergehenden Ausführung bezeichnen dabei gleiche Teile. Die obere, feststehende Scheibe weist bei diesem Beispiel lediglich einen Anschluss 29 für ein Massenspektrometer auf und es sind in der Scheibe 27 Kanäle 30 und 31 vorgesehen, welche beide konzentrischen Anschlusskreise mit dem einen Massenspektrometer auf kürzestem Wege verbinden, wobei auch hier die Verbindung infolge des winkelmässigen Versatzes der Anschlussstücke an der unteren Scheibe ein sequentielles Verbinden der jeweiligen Gasprobenleitungen mit dem Massenspektrometer erfolgt. Die Scheibe 27 wird anstelle einer normalen Scheibe mit zwei getrennten Kanälen und zwei Anschlussstutzen für zwei Massenspektrometer an dem Verteilkopf installiert und eines der beiden Massenspektrometer wird mit dem Anschlussstutzen 29 verbunden, während das andere Massenspektrometer gewartet werden kann. Nach der Wartung kann die Scheibe 27 wieder durch eine normale Scheibe mit zwei Kanälen (wie aus EP-A-0 579 952 bekannt) ersetzt werden. Figur 8 zeigt eine Ansicht der feststehenden Scheibe 27 von oben, worin die Kanäle 17, 18 und 19, 20 ersichtlich sind. Durch einen genügenden winkelmässigen Versatz der Anschlussstücke und verkürzte Kanäle 19, 20 kann sichergestellt werden, dass z.B. das Anschlussstück auf dem inneren Anschlusskreis den Kanal 20 erst dann erreicht, wenn das vorangehende Anschlussstück auf dem äusseren Anschlusskreis den Kanal 19 bereits verlassen hat. Auf diese Weise wird die gesteuerte Abfolge der Gasproben ohne deren Vermischung sichergestellt. An der Scheibe kann ein Blindanschlussstutzen 33 vorgesehen sein, um die überzählige Anschlussleitung zu dem einen, nicht verwendeten Massenspektrometer definiert parkieren zu können, so dass diese Leitung vor Verschmutzung geschützt ist. Anstelle der zwei zusammenlaufenden Kanäle 30 und 31 könnte in der Scheibe z.B. auch eine horizontal verlaufende Verbindung der Kanäle 19 und 20 vorgesehen sein, von welcher aus eine Verbindung zum Anschlussstutzen 29 vorgesehen wäre, was eine Anordnung ähnlich einem umgedrehten T ergäbe.

Anstelle von Massenspektrometern können natürlich auch andere Prüfgeräte zur Gasanalyse vorgesehen sein, z.B. Pulsfluoreszenzspektrometer.

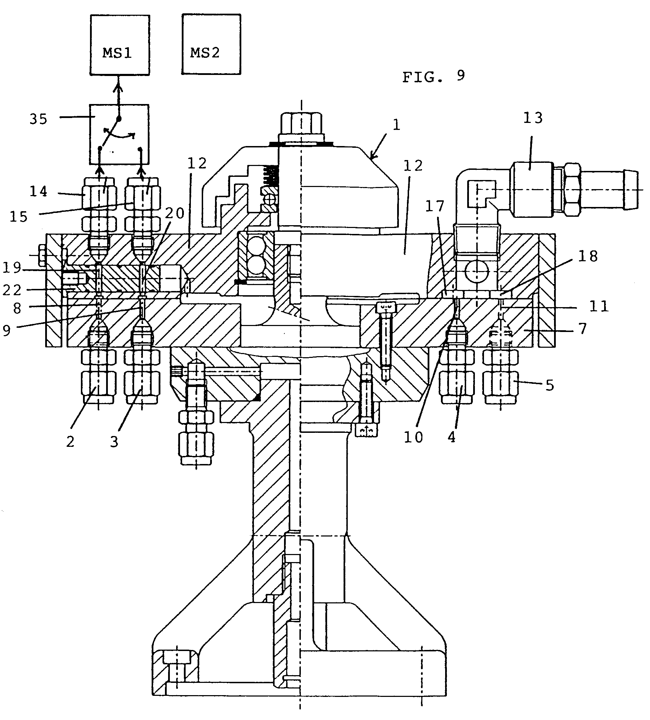

Figur 9 zeigt eine Variante der Erfindung, bei welcher anstelle eines Auswechselteils am Verteilkopf ein elektrisch steuerbares Ventil 35 für den Zeitraum der Reparatur oder Wartung des Prüfgerätes MS2 (zweites Massenspektrometer) zwischen die Anschlüsse 14 und 15 an der feststehenden Scheibe 12 und dem Prüfgerät MS1 (erstes Massenspektrometer) geschaltet ist. Dieses Ventil 35 wird also während der Reparatur- oder Wartungszeit montiert und nachfolgend wieder demontiert, analog der Lösung mit auswechselbarem Stopfen (dieser ist in Figur 9 trotzdem dargestellt, wird hier aber nicht benötigt; anstelle des Stopfens würde sich hier die feststehende Scheibe 12 bis zur beweglichen Scheibe 7 erstrecken). Das Umschalten der Gaswege erfolgt mit dem elektrisch umschaltbaren Ventil, welches die Gasproben von den Anschlüssen 2, 3 taktweise (getriggert) abwechslungsweise auf das funktionstüchtige Analysegerät MS1 umleitet. Anstelle des nur fallweise montierten Ventils 35 kann auch eine (nicht dargestellte) Ventilanordnung vorgesehen sein, welche dauernd an den Anschlüssen 14, 15 und den beiden Prüfgeräten angeschlossen ist, und welche durch das Umschalten von Ventilen die Gasproben wahlweise auf beide oder auf nur eines der beiden Prüfgeräte leiten kann.

## Patentansprüche

1. Vorrichtung zum Prüfen von entlang einer Karussell-Fördereinrichtung geförderten Behältern, insbesonders Flaschen, auf das Vorhandensein von Verunreinigungen, durch Prüfung von aus einzelnen Behältern entnommenen Gasproben, wobei einer Mehrzahl von Behältern gleichzeitig Gas entnommen wird und die entnommenen Gasproben durch eine Verteileinrichtung (1) in gesteuerter Reihenfolge nacheinander einer Prüfung zugeführt werden, wobei die Verteileinrichtung in der Drehachse des Karussells vorgesehen ist und eine feststehende Scheibe (12; 27) umfasst, welche mit einer Prüfeinrichtung und einem Saugmittel verbunden ist, und eine drehbare Scheibe (7) umfasst, an welcher in zwei konzentrischen Kreisen die Leitungen zu den Probeentnahmeorganen der Vorrichtung angeschlossen sind, dadurch gekennzeichnet, dass Verbindungskanäle (19,20; 23,24; 30,31) zwischen der beweglichen und der feststehenden Scheibe vorgesehen sind, deren Verlauf durch Auswechslung mindestens eines Teils der feststehenden Scheibe veränderbar ist, derart, dass die Verbindung von den in den zwei konzentrischen Kreisen angeordneten Anschlussstücken (2,3) wahlweise zu einem oder zu zwei Prüfgeräten der Prüfeinrichtung erfolgt.

2. Vorrichtung nach Anspruch 1, dadurch gekennzeichnet, dass die feststehende Scheibe (27) einen Anschluss (29) für ein Prüfgerät, insbesondere ein Massenspektrometer, und einen sich von diesem Anschluss zu den beiden Anschlussstücken hin verzweigenden Kanal (30,31) aufweist.

3. Vorrichtung nach Anspruch 1, dadurch gekennzeichnet, dass in der feststehenden Scheibe (12) ein auswechselbares Teil (22) vorgesehen ist, welches eine Kanalanordnung (19,20;23,24) zur wahlweisen Verbindung beider Anschlusskreise zu einem oder zu zwei Prüfgeräten, insbesondere Massenspektrometern, aufweist.

4. Vorrichtung nach Anspruch 3, dadurch gekennzeichnet, dass die Länge von in der feststehenden Scheibe angeordneten Ansaugkammern veränderbar ist, insbesondere mittels Drehschiebern (25,26,25',26').

5. Vorrichtung nach einem der Ansprüche 1 bis 4, dadurch gekennzeichnet, dass die Prüfeinrichtung eine Gasanalyseeinrichtung ist und insbesondere ein oder zwei Massenspektrometer und/oder Pulsfluoreszenzspektrometer aufweist.

6. Vorrichtung zum Prüfen von entlang einer Karussell-Fördereinrichtung geförderten Behältern, insbesonders Flaschen, auf das Vorhandensein von Verunreinigungen, durch Prüfung von aus einzelnen Behältern entnommenen Gasproben, wobei einer Mehrzahl von Behältern gleichzeitig Gas entnommen wird und die entnommenen Gasproben durch eine Verteileinrichtung (1) in gesteuerter Reihenfolge nacheinander einer Prüfung zugeführt werden, wobei die Verteileinrichtung in der Drehachse des Karussells vorgesehen ist und eine feststehende Scheibe (12; 27) umfasst, welche mit einer Prüfeinrichtung und einem saugmittel verbunden ist, und eine drehbare Scheibe (7) umfasst, an welcher in zwei konzentrischen Kreisen die Leitungen zu den Probeentnahmeorganen der Vorrichtung angeschlossen sind, dadurch gekennzeichnet, dass zwischen den Anschlüssen (14, 15) zum Anschluss der Prüfeinrichtung an der feststehenden Scheibe und der Prüfeinrichtung ein elektrisch steuerbares Umschaltmittel vorgesehen ist, welches Umschaltmittel die Verbindung von den in den zwei konzentrischen Kreisen angeordneten Anschlussstücken (2, 3) wahlweise zu nur einem von zwei Prüfgeräten der Prüfeinrichtung oder zu beiden Prüfgeräten vornimmt.

7. Vorrichtung nach Anspruch 6, dadurch gekennzeichnet, dass die Prüfeinrichtung eine Gasanalyseeinrichtung ist und insbesondere ein oder zwei Massenspektrometer und/oder Pulsfluoreszenzspektrometer aufweist.
